# EUROPEAN PATENT APPLICATION

(11) **EP 4 193 969 A1**
(43) Date of publication of application: **14.06.2023**
(21) Application number: 21213595.8
(22) Date of filing: 10.12.2021
(51) Int. Cl.: A61F 2/78, A61F 2/28

(54) **SYSTEM OF INTRA-BONE PROSTHESIS FOR AMPUTATION**

(71) Applicant: Arthemidex S.r.l., 20123 Milano (IT)
(72) Inventor: MARTINI, Filippo Maria, 20123 Milano (IT); RAGONE, Vincenza Anna, 20123 Milano (IT); AROSIO, Massimo, 20123 Milano (IT)
(74) Representative: Long, Giorgio

(57) **Abstract**

The present invention relates to an intraosseous transcutaneous prosthesis system adapted to be applied to amputated limbs, in particular for veterinary use.

In particular, the invention relates to an intramedullary bone fixation system (1) of an amputation prosthesis, comprising:
a) an intramedullary stem (2),
b) a percutaneous element (3) configured to removably couple with the intramedullary stem (2),
c) a bone fixation element (4) configured to removably couple between said intramedullary stem (2) and said percutaneous element (3), and
d) a retention cuff (5),

in which elements a), b), c) and d) are separate elements so as to create a modular system.

## Description

The present invention relates to an intraosseous transcutaneous prosthesis system adapted to be applied to amputated limbs, in particular for veterinary use.

### Background art

The use of intraosseous transcutaneous amputation prostheses (ITAP) in association with partial limb amputations is a new technique for the treatment of appendicular neoplasia, irreversible vascular or neurological compromise or severe trauma in pets.

The development of this strategy rather than the choice of total amputation has become necessary because, although dogs and cats are able to tolerate walking with three legs well, they can develop gait alterations and musculoskeletal problems, as well as pain. This adds to the increased sensitivity of owners who want to offer their pets the best possible care and quality of life.

ITAP allows connecting an intramedullary metal implant to an exoprosthesis, by virtue of a transcutaneous pin. Thereby the load forces are transferred directly from the artificial foot to the appendicular skeleton according to natural patterns, allowing similar-to-normal muscle development. Furthermore, the classic problems associated with grip prostheses are overcome, such as erosions, ulcers, skin breaks or limitations due to poor compliance of the patient or owner.

However, even this technique is not free from limits and complications, first of all infections, as the transcutaneous pin breaks the epithelial barrier and can be the vehicle of pathogens towards the bone and the surrounding soft tissues. To overcome this problem, a natural biomimetic model was observed and analyzed, the deer's antler, which led to the creation of a porous flange to be inserted immediately below the epidermis.

Ideally, it would be necessary to optimize the three systems: bone, skin and implant and the related interfaces thereof, which by virtue of the synergistic action thereof ensure stability to the implant and the creation of a biologically stable and safe seal.

A further problem of the known ITAP implants lies in that they must be customized according to the segment of the concerned limb and the weight of the animal. This implies long times and high costs.

Therefore, it is an object of the invention to provide an intramedullary bone fixation system which is performing, in the sense of being provided with high compliance for the animal, versatile, and therefore of lower cost.

### Summary of the invention

The above object and others which may appear later are achieved by an intramedullary bone fixation system of an amputation prosthesis as outlined in the appended claims, the text of which forms an integral part of the present description.

In particular, the invention relates to an intramedullary bone fixation system of an amputation prosthesis, comprising:
a) an intramedullary stem,
b) a percutaneous element configured to removably couple with the intramedullary stem,
c) a bone fixation element configured to removably couple between said intramedullary stem and said percutaneous element, and
d) a retention cuff,
in which elements a), b), c) and d) are separate elements so as to create a modular system.

Further aspects of the invention are indicated in the following points:
2) A system as defined above, in which the intramedullary stem comprises an intramedullary element, configured to fix itself inside the medullary hole of the bone to which the prosthesis will be fixed and comprising a proximal end and a distal end, and a coupling element, configured to couple with the percutaneous element, an abutment flange being placed between said intramedullary element and said coupling element.
3) A system as in point 2, in which the intramedullary element has a smaller diameter than the coupling element and has a rounded concave profile at the distal end, in the junction point with the abutment flange.
4) A system as in point 2 or 3, in which the proximal end of the intramedullary element comprises a longitudinal hole having a non-circular edge, preferably hexagonal, to allow the insertion of an Allen key so as to allow fixing the intramedullary stem to the percutaneous element.
5) A system as in any one of points 2 to 4, in which the intramedullary element comprises a distal portion having a trabecular surface which preferably continues on at least one part of the abutment flange.
6) A system as in point 5, in which the trabecular surface has a porous and wrinkled structure and preferably consists of a grid of regular or random arrangement which by means of the connection of the various segments forms pores of variable size from 0.4 to 1.5 mm.
7) A system as in any one of points 2 to 6, in which the coupling element has a cylindrical threaded surface to allow the screw coupling of the intramedullary stem with the percutaneous element, and in which the percutaneous element comprises a proximal end and a distal end, in which the proximal end comprises a threaded longitudinal hole configured to screw couple with the coupling element of the intramedullary stem.
8) A system as in any one of points 1 to 7, in which the percutaneous element comprises a proximal end, and in which the percutaneous element comprises, at a preset distance from the proximal end, an abutment flange, the abutment flange comprising a front edge comprising a recess.
9) A system as in any one of points 1 to 8, in which the percutaneous element comprises first superficial grooves, configured to favor the coupling and fixation of the exoprosthesis of the limb or the single foot, and second superficial grooves, configured for the coupling and fixation of the retention cuff.
10) A system as in any one of points 2 to 9, in which the bone fixation element comprises a coupling ring with the intramedullary stem and with the percutaneous element and a fixation plate extending longitudinally from an edge portion of the coupling ring in the proximal direction, the length extension of the fixation plate being greater than the length extension of the intramedullary element of the intramedullary stem and comprising, close to a proximal end, through holes configured to allow the fixation with fixation screws to the external surface of the bone.
11) A system as in point 10, in which the bone fixation element comprises a tooth protruding from the coupling ring in the distal direction.
12) A system as in point 10 or 11, in which the fixation plate comprises, close to a distal end, a slot the bottom of which forms a hinge element which allows a bending of the fixation plate with respect to the remaining part of the fixation system, in which a shock-absorbing elastic element, such as a cup spring, is preferably arranged in said slot.
13) A system as in any one of points 1 to 12, in which the retention cuff comprises a disc having a central hole, the central hole having a diameter substantially corresponding to the diameter of the percutaneous element.
14) A system as in point 13, in which the disc has a plurality of micro-holes preferably covering at least 50% of the disc surface, or in which the disc is made of a porous material and preferably consists of a grid of regular or random arrangement which, through the connection of the various segments, forms pores of variable size from 0.4 to 1.5 mm.
15) A system as in any one of points 1 to 14, in which said system is made of biocompatible titanium alloy, preferably by 3D printing, and preferably contains antimicrobial agents such as copper or silver powder.

Further features and advantages will become apparent from the description of preferred, but not exclusive, embodiments of the invention, illustrated by way of non-limiting example.

### Brief description of the drawings

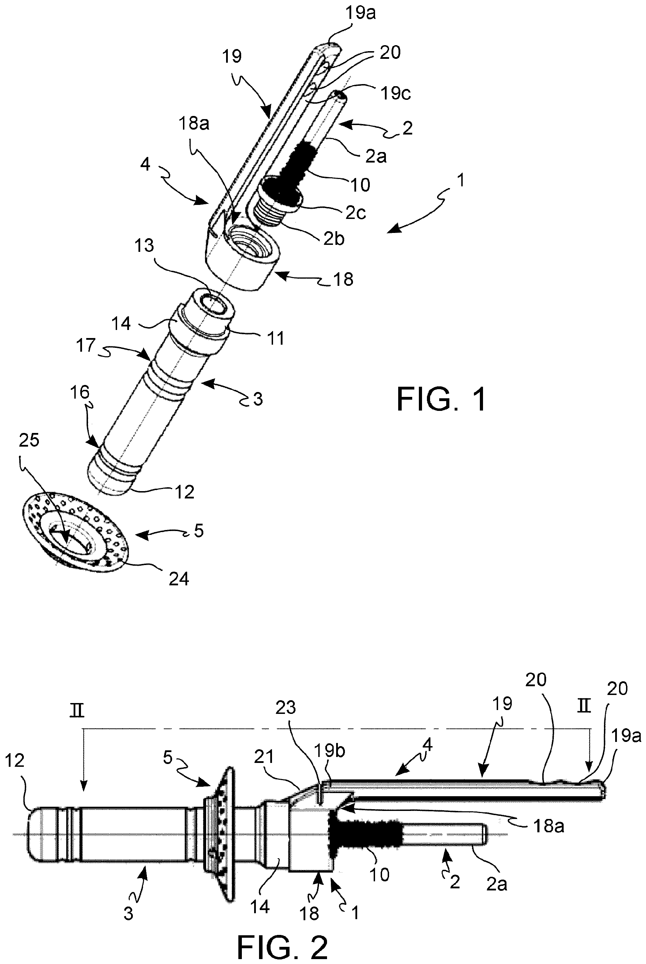

Figure 1 depicts an exploded perspective view of the fixation system according to the invention;
Figure 2 depicts a side view of the system in figure 1 in the assembled condition;

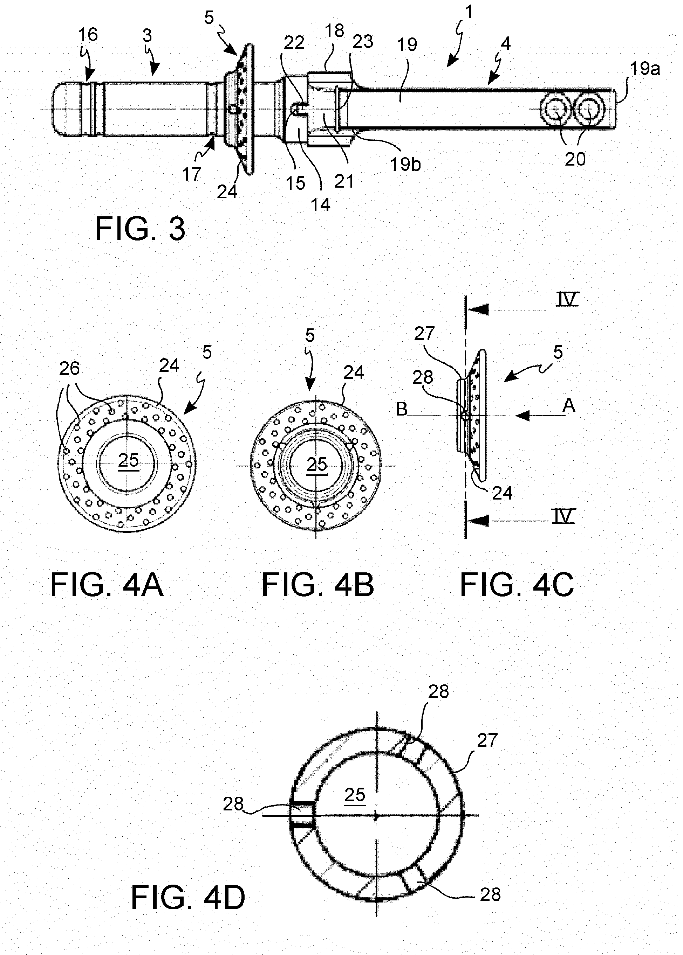

Figure 3 depicts a view of the fixation system according to the direction II-II in figure 2;
Figures 4A and 4B depict plan views, according to directions A and B, respectively, of a first detail of the fixation system of the invention;
Figure 4C depicts a side view of the detail in figures 4A-4B;
Figure 4D depicts a view according to the section IV-IV in figure 4C;

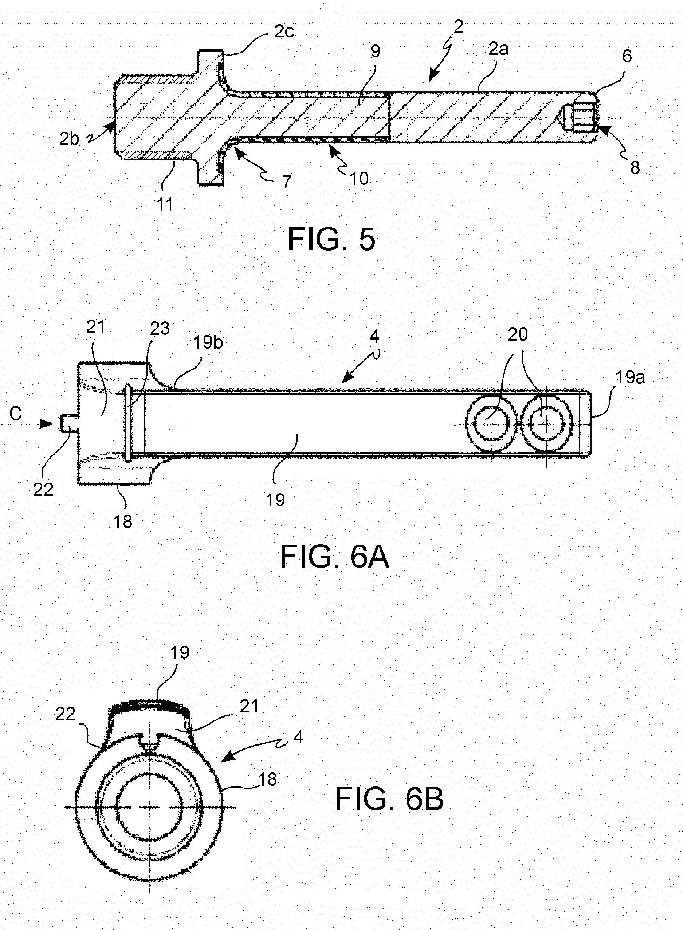

Figure 5 depicts a longitudinal section view of a second detail of the fixation system of the invention;
Figure 6A depicts a plan view according to the direction II-II in figure 2 of a third detail of the fixation system of the invention;
Figure 6B depicts a view according to the direction C in figure 6B;

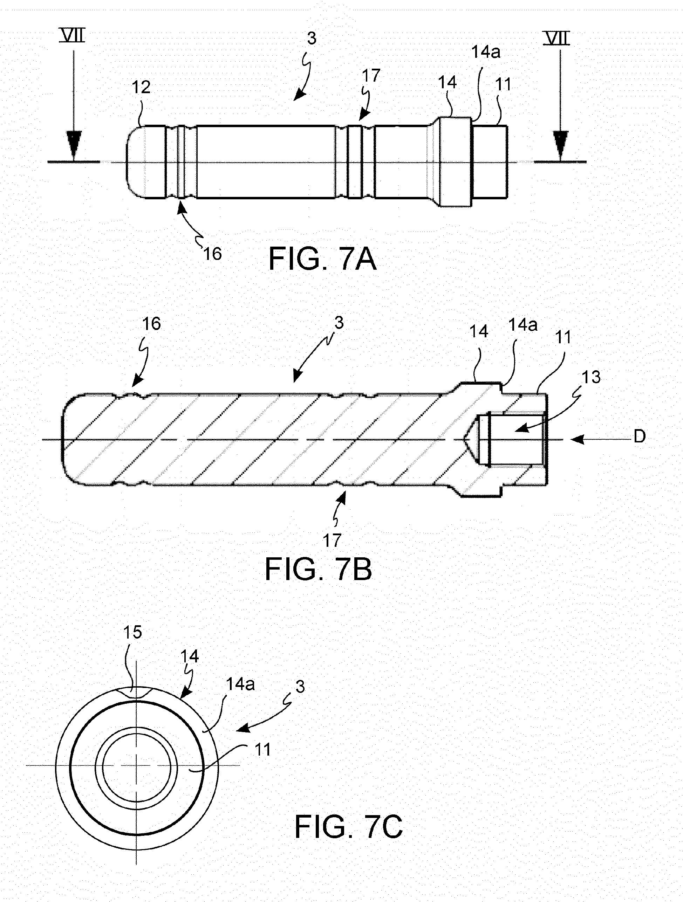

Figure 7A depicts a side view of a fourth detail of the fixation system of the invention;
Figure 7B depicts a view according to the section VII-VII in figure 7A;
Figure 7C depicts a view according to the direction D in figure 7B.

### Detailed description of the invention

With reference to the drawings, the intramedullary bone fixation system of an amputation prosthesis, indicated as a whole by numeral 1, is a modular system comprising:
- an intramedullary stem 2,
- a percutaneous element 3 configured to removably couple with the intramedullary stem 2,
- a bone fixation element 4 configured to removably couple between said intramedullary stem 2 and said percutaneous element 3, and
- a retention cuff 5.

The invention will now be described in more detail, again with reference to the accompanying drawings.

The intramedullary stem 2 can be implanted in the medullary canal and forms the connecting element between a long bone of the amputated limb and the external prosthesis through the percutaneous element 3.

The intramedullary stem 2 has a substantially cylindrical shape and comprises an intramedullary element 2a, configured to be fixed inside the medullary hole of the bone to which the prosthesis will be fixed, and a coupling element 2b, configured to couple with the percutaneous element 3. An abutment flange 2c is placed between the medullary element 2a and the coupling element 2b.

The intramedullary element 2a comprises a proximal end 6 and a distal end 7.

"Proximal end", according to the meaning adopted in the present invention, means the end closest to the patient's bone to which the prosthesis is to be fixed, while "distal end" means the opposite end.

In the embodiment shown in the figures, the intramedullary element 2a has a smaller diameter than the coupling element 2b and has a rounded concave profile at the distal end 7 at the junction point with the abutment flange 2c.

The proximal end 6 of the intramedullary element 2a comprises a longitudinal hole 8 having a non-circular edge, typically hexagonal, to allow the insertion of an Allen key so as to allow fixing the intramedullary stem 2 to the percutaneous element 3.

The medullary element 2a comprises a distal portion 9 having a trabecular surface 10 which continues on at least one part of the abutment flange 2c.

The trabecular surface 10 has a porous and wrinkled structure and has the function of promoting and accelerating bone regrowth after implantation.

In preferred embodiments, the trabecular surface 10 consists of a grid of regular or random arrangement which by means of the connection of the various segments forms pores of variable size from 0.4 to 1.5 mm.

The coupling element 2b has a cylindrical threaded surface 11 to allow the screw coupling of the intramedullary stem 2 with the percutaneous element 3.

The percutaneous element 3 comprises a substantially cylindrical body and has a proximal end 11 and a distal end 12, in which the proximal end 11 comprises a longitudinal threaded hole 13 configured to screw couple with the coupling element 2b of the intramedullary stem 2.

At a predetermined distance from the proximal end 11, the percutaneous element comprises an abutment flange 14. Said predetermined distance, as will be better clarified in the following, is determined to allow the sandwich coupling, between the intramedullary stem 2 and the percutaneous element 3, of the bone fixation element 4.

The abutment flange 14 of the percutaneous element 3 comprises a front edge 14a comprising a recess 15 the function of which will be explained below.

Close to the distal end 12, the percutaneous element 3 comprises first superficial grooves 16 configured to favor the coupling and fixation of the exoprosthesis of the limb or the single foot.

Second surface grooves 17 are placed on the percutaneous element 3 between the first surface grooves 16 and the abutment flange 14, configured for the coupling and fixation of the retention cuff 5.

The bone fixation element 4 allows partially transferring the load from the intramedullary element 2a of the intramedullary stem 2 to the percutaneous element 3.

The bone fixation element 4 comprises a coupling ring 18 with the intramedullary stem 2 and with the percutaneous element 3 and a fixation plate 19 extending longitudinally from an edge portion 18a of the coupling ring 18 in the proximal direction, i.e., in the direction opposite the direction along which the percutaneous element 3 extends.

The length extension of the fixation plate 19 is greater than the length extension of the intramedullary element 2a of the intramedullary stem 2 and comprises, close to the proximal end 19a, through holes 20 (specifically, in the embodiment shown, two through holes 20), so as to allow the fixation with fixing screws to the external surface of the bone.

The fixation plate 19 comprises a lower or internal surface 19c arched in shape, so as to adapt to the shape of the external surface of the bone to which it is to be fixed.

At the distal end 19b, the fixation plate 19 comprises an inclined profile 21 which ends with a tooth 22 protruding from the coupling ring 18 in the distal direction. The tooth 22 has a shape and dimensions such as to be inserted in the recess 15 of the percutaneous element 3, so as to prevent the relative rotation of the bone fixation element 4 with respect to the intramedullary stem 2 and the percutaneous element 3.

The fixation plate 19, close to the distal end 19b thereof, preferably at the junction point between the fixation plate 19 and the inclined profile 21, comprises a slot 23 the bottom of which forms a hinge element which allows a bending of the fixation plate 19 with respect to the remaining part of the fixation system 1. This allows avoiding the passage of load between the fixation screws inserted in the through holes 20 and the percutaneous element 3 and to prevent the load from transferring directly from the proximal portion of the fixation plate 19 to the proximal portion of the percutaneous element 3 while maintaining the intramedullary stem 2 unloaded, thus risking to prevent bone integration.

In certain embodiments, said slot 23 can house a shock-absorbing elastic element, such as a cup spring.

The coupling ring 18 is configured to fit and sandwich between the stop flange 2c of the intramedullary stem 3 and the front surface 14a of the stop flange 14 of the percutaneous element 3, when these are assembled by screwing, so as to insert the tooth 22 in the recess 15.

The retention cuff 5 comprises a conical disc 24 having a central hole 25. The central hole 25 has a diameter substantially corresponding to the diameter of the percutaneous element 3 and is configured to couple with the second surface grooves 17 of the percutaneous element 3.

The conical disc 24 has a plurality of micro-holes 26 and is configured to retain the emerging soft tissues and to support the suture of the skin. It can be made of solid material or porous material.

In certain embodiments, the micro-holes 26 cover at least 50% of the surface of the conical disc 24.

In certain embodiments, the porous material consists of a grid of regular or random arrangement which, through the connection of the various segments, forms pores of variable size from 0.4 to 1.5 mm.

A sleeve portion 27 extends distally from the central hole 25 and comprises transverse holes 28 for fixing the retention cuff 5 to the percutaneous element 3 by means of fixing screws.

The fixation system 1 of the invention is made of titanium alloy, so as to be biocompatible and easily integrable in tissues.

The fixation system according to the invention is preferably made by 3D printing. With such a technique it is also possible to directly obtain the trabecular surface 10 described above.

In certain embodiments, the titanium alloy can contain antimicrobial agents, such as copper or silver powder.

The system of the invention allows a total modulation. In fact, all the parts forming it, i.e., the intramedullary stem, the percutaneous element, the bone fixation element and the retention cuff, can be made in different shapes and sizes and can be combined according to various needs. Therefore, it is not necessary to create a customized fixation system as with the prior art systems.

It is apparent that only some particular embodiments of the present invention have been described, to which those skilled in the art will be able to make all changes required to adapt it to particular applications, without departing from the scope of protection of the present invention.

In practice, the materials used, as long as they are compatible with the specific use, as well as the contingent shapes and dimensions, could be varied according to requirements and the advancement of the art.

## Claims

1. An intramedullary bone fixation system (1) of an amputation prosthesis, comprising:
a) an intramedullary stem (2),
b) a percutaneous element (3) configured to removably couple with the intramedullary stem (2),
c) a bone fixation element (4) configured to removably couple between said intramedullary stem (2) and said percutaneous element (3), and
d) a retention cuff (5),
wherein elements a), b), c) and d) are separate elements so as to create a modular system.

2. The system (1) according to claim 1, wherein the intramedullary stem (2) comprises an intramedullary element (2a), configured to fix itself inside the medullary hole of the bone to which the prosthesis will be fixed and comprising a proximal end (6) and a distal end (7), and a coupling element (2b), configured to couple with the percutaneous element (3), an abutment flange (2c) being placed between said intramedullary element (2a) and said coupling element (2b).

3. The system (1) according to claim 2, wherein the intramedullary element (2a) has a smaller diameter than the coupling element (2b) and has a rounded concave profile at the distal end (7), at the junction point with the abutment flange (2c).

4. the system (1) according to claim 2 or 3, wherein the proximal end (6) of the intramedullary element (2a) comprises a longitudinal hole (8) having a non-circular edge, preferably hexagonal, to allow the insertion of an Allen key so as to allow fixing the intramedullary stem (2) to the percutaneous element (3).

5. The system (1) according to any one of claims 2 to 4, wherein the intramedullary element (2a) comprises a distal portion (9) having a trabecular surface (10) which preferably continues on at least one part of the abutment flange (2c).

6. The system (1) according to claim 5, wherein the trabecular surface (10) has a porous and wrinkled structure and preferably consists of a grid of regular or random arrangement which through the connection of the various segments forms pores of variable size from 0.4 to 1.5 mm.

7. the system (1) according to any one of claims 2 to 6, wherein the coupling element (2b) has a cylindrical threaded surface (11) to allow the screw coupling of the intramedullary stem (2) with the percutaneous element (3), and wherein the percutaneous element (3) comprises a proximal end (11) and a distal end (12), wherein the proximal end (11) comprises a threaded longitudinal hole (13) configured to screw couple with the coupling element (2b) of the intramedullary stem (2).

8. The system (1) according to any one of claims 1 to 7, wherein the percutaneous element (3) comprises a proximal end (11), and wherein the percutaneous element (3) comprises, at a preset distance from the proximal end (11), an abutment flange (14), the abutment flange (14) comprising a front edge (14a) comprising a recess (15).

9. The system (1) according to any one of claims 1 to 8, wherein the percutaneous element (3) comprises first surface grooves (16), configured to favor the engagement and fixation of the exoprosthesis of the limb or the single foot, and second surface grooves (17), configured for the coupling and fixation of the retention cuff (5).

10. The system (1) according to any one of claims 2 to 9, wherein the bone fixation element (4) comprises a coupling ring (18) with the intramedullary stem (2) and with the percutaneous element (3) and a fixation plate (19) extending longitudinally from an edge portion (18a) of the coupling ring (18) in the proximal direction, the length extension of the fixation plate (19) being greater than the length extension of the intramedullary element (2a) of the intramedullary stem (2) and comprising, close to a proximal end (19a), through holes (20) configured to allow the fixation with fixing screws to the external surface of the bone.

11. The system (1) according to claim 10, wherein the bone fixation element (4) comprises a tooth (22) protruding from the coupling ring (18) in the distal direction.

12. The system (1) according to claim 10 or 11, wherein the fixation plate (19) comprises, close to a distal end (19b), a slot (23) the bottom of which forms a hinge element which allows a bending of the fixation plate (19) with respect to the remaining part of the fixation system (1), wherein a shock-absorbing elastic element, such as a cup spring, is preferably arranged in said slot (23).

13. The system (1) according to any one of claims 1 to 12, wherein the retention cuff (5) comprises a disc (24) having a central hole (25), the central hole (25) having a diameter substantially corresponding to the diameter of the percutaneous element (3).

14. The system (1) according to claim 13, wherein the disc (24) has a plurality of micro-holes (26) which preferably cover at least 50% of the surface of the disc (24), or wherein the disc (24) is made of a porous material and preferably consists of a grid of regular or random arrangement which by means of the connection of the various segments forms pores of variable size from 0.4 to 1.5 mm.

15. The system (1) according to any one of claims 1 to 14, wherein said system (1) is made of biocompatible titanium alloy, preferably by 3D printing, and preferably contains antimicrobial agents such as copper or silver powder.
